# EUROPEAN PATENT SPECIFICATION

(11) **EP 0 726 778 B1**
(45) Date of publication and mention of the grant of the patent: **25.07.2001**
(21) Application number: 95911690.6
(22) Date of filing: 08.02.1995
(51) Int. Cl.: A61K 47/48

(54) **ORAL DELIVERY SYSTEM OF CHEMICALLY MODIFIED PROTEINS G-CSF**
ORALES VERABREICHUNGSSYSTEM VON CHEMISCHMODIFIZIERTEN PROTEINEN G-CSF
SYSTEME D'ADMINISTRATION PAR VOIE ORALE DE PROTEINES G-CSF MODIFIEES CHIMIQUEMENT

(30) Priority: 08.02.1994 US 194187; 01.02.1995 US 379121
(43) Date of publication of application: 21.08.1996
(60) Divisional application: 00122148.0
(73) Proprietor: Amgen Inc., Thousand Oaks, California 91320-1799 (US)
(72) Inventor: HABBERFIELD, Alan D., Pacific Palisades, CA 90272 (US)
(74) Representative: Vossius, Volker, Dr.
(86) International application number: US9501752
(87) International publication number: WO9521629

(56) References cited:
- EP-A- 0 401 384
- EP-A- 0 452 179
- EP-A- 0 473 268
- EP-A- 0 576 192
- EP-A- 0 593 868
- WO-A-90/01329
- WO-A-93/21229
- WO-A-94/02164
- WO-A-94/20069
- JOURNAL OF CONTROLLED RELEASE, vol. 30, no. 1, April 1994 AMSTERDAM NL, pages 27-34,

### SEQUENCE LISTING

### (1) GENERAL INFORMATION:

(i) APPLICANT: Amgen Inc.
(ii) TITLE OF INVENTION: Oral Delivery of Chemically Modified Proteins
(iii) NUMBER OF SEQUENCES: 2
(iv) CORRESPONDENCE ADDRESS:
   (A) ADDRESSEE: Amgen Inc.
   (B) STREET: 1840 Dehavilland Drive
   (C) CITY: Thousand Oaks
   (D) STATE: California
   (E) COUNTRY: USA
   (F) ZIP: 91320-1789
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER: US 08/194,187
   (B) FILING DATE: 02-FEB-1994
   (C) CLASSIFICATION:
(viii) ATTORNEY/AGENT INFORMATION:
   (A) NAME: Pessin, Karol M.
   (C) REFERENCE/DOCKET NUMBER: A-285

### (2) INFORMATION FOR SEQ ID NO:1:

(i) SEQUENCE CHARACTERISTICS:
   (A) LENGTH: 531 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:1:
   ATGACTCCAT TAGGTCCTGC TAGCTCTCTG CCGCAAAGCT TTCTGCTGAA ATGTCTGGAA      60
   CAGGTTCGTA AAATCCAGGG TGACGGTGCT GCACTGCAAG AAAAACTGTG CGCTACTTAC     120
   AAACTGTGCC ATCCGGAAGA GCTGGTACTG CTGGGTCATT CTCTTGGGAT CCCGTGGGCT     180
   CCGCTGTCTT CTTGTCCATC TCAAGCTCTT CAGCTGGCTG GTTGTCTGTC TCAACTGCAT     240
   TCTGGTCTGT TCCTGTATCA GGGTCTTCTG CAAGCTCTGG AAGGTATCTC TCCGGAACTG     300
   GGTCCGACTC TGGACACTCT GCAGCTAGAT GTAGCTGACT TTGCTACTAC TATTTGGCAA     360
   CAGATGGAAG AGCTCGGTAT GGCACCAGCT CTGCAACCGA CTCAAGGTGC TATGCCGGCA     420
   TTCGCTTCTG CATTCCAGCG TCGTGCAGGA GGTGTACTGG TTGCTTCTCA TCTGCAATCT     480
   TTCCTGGAAG TATCTTACCG TGTTCTGCGT CATCTGGCTC AGCCGTAATA G              531

### (2) INFORMATION FOR SEQ ID NO:2:

(i) SEQUENCE CHARACTERISTICS:
   (A) LENGTH: 175 amino acids
   (B) TYPE: amino acid
   (C) STRANDEDNESS: single
   (D) TOPOLOGY: linear
(ii) MOLECULE TYPE: protein
(xi) SEQUENCE DESCRIPTION: SEQ ID NO:2:
   Met Thr Pro Leu Gly Pro Ala Ser Ser Leu Pro Gln Ser Phe Leu Leu
   1               5                   10                  15
   Lys Cys Leu Glu Gln Val Arg Lys Ile Gln Gly Asp Gly Ala Ala Leu
               20                  25                  30
   Gln Glu Lys Leu Cys Ala Thr Tyr Lys Leu Cys His Pro Glu Glu Leu
           35                  40                  45
   Val Leu Leu Gly His Ser Leu Gly Ile Pro Trp Ala Pro Leu Ser Ser
       50                  55                  60
   Cys Pro Ser Gln Ala Leu Gln Leu Ala Gly Cys Leu Ser Gln Leu His
   65                  70                  75                  80
   Ser Gly Leu Phe Leu Tyr Gln Gly Leu Leu Gln Ala Leu Gln Gly Ile
                   85                  90                  95
   Ser Pro Glu Leu Gly Pro Thr Leu Asp Thr Leu Gln Leu Asp Val Ala
               100                 105                 110
   Asp Phe Ala Thr Thr Ile Trp Gln Gln Met Glu Glu Leu Gly Met Ala
           115                 120                 125
   Pro Ala Leu Gln Pro Thr Gln Gly Ala Met Pro Ala Phe Ala Ser Ala
       130                 135                 140
   Phe Gln Arg Arg Ala Gly Gly Val Leu Val Ala Ser His Leu Gln Ser
   145                 150                 155                 160
   Phe Leu Glu Val Ser Tyr Arg Val Leu Arg His Leu Ala Gln Pro
                   165                 170                 175

## Description

### Field of the Invention

The present invention relates to novel compositions for the oral delivery of chemically modified proteins. (The term "protein is here used interchangeably with the term "polypeptide" unless otherwise indicated). Further, the present invention relates to novel compositions and methods for the oral delivery of pegylated proteins. In another aspect, the present invention relates to novel compositions and methods for oral delivery of chemically modified granulocyte colony stimulating factor (G-CSF), and, in yet another aspect, particularly, oral delivery of pegylated G-CSF.

### Background

Currently, injection is the typical mode of administering a biologically active protein to the blood stream. Injection, however, is undesireable in many instances. The recipient, of course, may experience discomfort or pain, and may have to travel to a trained practitioner for the injection. For these reasons and others there may be problems with patient compliance using injection as a mode of administration. One alternative to injection is the oral administration of biologically active proteins.

Oral administration has been problematic, however, for a variety of reasons. One major concern is the degradation of the biologically active protein in the gut. Protease inhibitors have been proposed. There have also been various pharmaceutical preparations of oral dosage forms for various proteins which protect the protein from degradation, e.g., EP 0 459 795, entitled "Oral dosage form of biologically active proteins," (see also, US Patent US-A-5 597 562, entitled, Oral Dosage Form of Biologically Active Proteins).

U.S. Patent No. 4,925,673 (Steiner et al.), entitled, "Delivery Systems for Pharmacological Agents Encapsulated with Proteinoids" reports the oral delivery of insulin, heparin and physostigmine encapsulated in certain microspheres which are predominantly less than about 10 microns in diameter. These proteinoids are made of an acidic protein that is reportedly stable in the presence of stomach enzymes and acid, but which release the microencapsulated agent in the near neutral blood stream. There has also been a report of the use of this microsphere for oral delivery of a monoclonal antibody.

Other groups have attempted to increase oral uptake of therapeutics by their incorporation into polystyrene latex nanoparticles and microparticles. Thus the drug is not only protected from the hostile environment but also these particles are then taken up from the enteral route into the systemic circulation via the Peyers patches. See Jani et al., J. Pharm. Pharmacol. 42: 821-826 (1990), see also, Jani et al., Intl J. Pharm. 86: 239-246 (1992).

Using a similar approach for both the protection and enhanced uptake of the peptide or protein, microemulsions have been claimed for the oral delivery of such therapeutics as insulin, calcitonin and somatotrophin or growth factors. PCT Publication No. WO 90/03164. Additionally, the oral delivery of therapeutics using liposomes has been investigated, see Aramaki et al., Pharm. Res. 10: 1228-1231 (1993). The liposomes were composed of distearoylphosphadtidylcholine, phosphatidylserine, and cholesterol or dipalmitoylphosphatidylcholine, phosphatidylserine and cholesterol which were stable in the gut and appeared to be taken up by the Peyers patches in the lower ileum. To date, despite the above reports, oral dosage forms of biologically active proteins are not widely in clinical use.

This may be attributable to the technical hurdles involved in attempting to deliver a therapeutic protein into the systemic circulation from the oral route. Briefly, the digestive process is, by definition, hostile to any ingested protein. The gastrointestinal tract is an organ developed to both physically and chemically break down ingested nutrients and is responsible for their uptake into the body and for the elimination of waste. Ingested food is immediately degraded in the stomach by the combination of low pH, typically 1-3 (Dotevall, G., et al. Acta Med. Scand., 170, 59. 1961) and strong peristaltic contractions which maintain the nutrients in the stomach while continuing to physically break down the food. In addition the protease pepsin is secreted into the lumen of the stomach from the gastric chief cells. The result of this extremely hostile environment is that the food is eventually released into the small intestine, specifically the duodenum, through the pylorus as small particles of ~1 mm or less (Mayer, E.A., et al. Gastroenterology, 87, 1264-1271, 1984). The pH of the stomach contents entering the duodenum is rapidly elevated to pH 5-7 by bicarbonate in the bile and pancreatic secretions. Additionally, the endoproteases trypsin, chymotrypsin and elastase are released into the duodenal lumen along with many enzymes for the digestion of polysaccharides and lipids. The products of these proteases are generally small peptides and these in turn are hydrolyzed to amino acids prior to absorption by exopeptidases in the brush border of the enterocytes lining the intestine (for reviews see Kenny, A.J. and Fulcher, I.S., In: *Brush Border Membranes,* edited by R. Porter and G. M. Collins, pp 12-33, 1983 and Tobey, N., et al. Gastroenterology, 88;. 913-926 (1985). Proteolysis, and more general digestion of the food takes place throughout the small intestine, i.e. the duodenum, jejunum and ileum, as does uptake of the products of digestion. The functions of the large intestine, which consists of the caecum and the colon, are water and electrolyte extraction from the lumen into the body, and storage and eventual elimination of waste.

The products of digestion are generally absorbed through active uptake processes for amino acids and for monosacchorides, while others, specifically lipids, are absorbed by a more passive diffusion process into the enterocytes lining the gut. Active uptake processes are also known to exist for some vitamins and other larger but essential nutritive factors which are unable to be passively absorbed. However, for most large molecules the enterocyte lining of the gut lumen is an inpenetrable barrier which cannot be crossed.

Throughout the gut, the enterocyte lining of the intestine absorbs digestion products. Large molecules, such as those of greater than about 500-1000 Da MW, are not known to be passively absorbed by the intestine.

Therefore, the art teaches against enlarging the size of a biologically active protein for oral administration. For example, polyethylene glycol alone is thought to pass through the intestinal tract with little or no absorbance, Ma et al., Gastroenterology 98: 39-46 (1990); Sundquist et al., Gut 21: 208-214 (1980).

One such biologically active protein, which is the subject of the examples below, is granulocyte colony stimulating factor, "G-CSF." G-CSF promotes the formation from bone marrow cells of certain bacteria-fighting white blood cells, called neutrophilic granulocytes, or "neutrophils." Once released into the circulating blood, neutrophilic granulocytes enable the human immune system to ward off bacterial infection. G-CSF induces the rapid proliferation and release of neutrophilic granulocytes to the blood stream.

Human G-CSF can be obtained and purified from a number of sources. Natural human G-CSF (nhG-CSF) can be isolated from the supernatants of cultured human tumor cell lines. The recombinant production of G-CSF enabled sufficient amounts of G-CSF with desired therapeutic qualities (recombinant production is described in U.S. Patent No. 4,810,643 (Souza). Recombinant human G-CSF (rhG-CSF) has been successfully used in the clinic for restoration of immune function after chemotherapy and radiation therapy, and in chronic settings, such as severe chronic neutropenia. Presently, the recombinant human G-CSF (generic name, Filgrastim) is sold commercially in the United States under the brand name Neupogen®, and is administered by injection or infusion.

Proteins may be protected against proteolysis by the attachment of chemical moieties. Such attachment may effectively block the proteolytic enzyme from physical contact with the protein backbone itself, and thus prevent degradation. Polyethylene glycol is one such chemical moiety which has been shown to protect against proteolysis. Sada, et al., J. Fermentation Bioengineering 71: 137-139 (1991).

In addition to protection against proteolytic cleavage, chemical modification of biologically active proteins has been found to provide additional advantages under certain circumstances, such as increasing the stability and circulation time of the therapeutic protein and decreasing immunogenicity. See U.S. Patent No. 4,179,337, Davis et al., issued December 18, 1979. For a review, see Abuchowski et al., in Enzymes as Drugs. (J.S. Holcerberg and J. Roberts, eds. pp. 367-383 (1981)). A review article describing protein modification and fusion proteins is Francis, *Focus on Growth Factors* 3: 4-10 (May 1992) (published by Mediscript, Mountview Court, Friern Barnet Lane, London N20, OLD, UK). For example, see EP 0 401 384, entitled, *"Chemically modified Granulocyte Colony Stimulating Factor,"* which describes materals and methods for preparing G-CSF to which polyethylene glycol molecules are attached. The addition of polyethylene glycol increases stability of G-CSF at physiological pH as compared to non-pegylated G-CSF (such modified G-CSF is referred to herein as "pegylated G-CSF" or "PEG-G-CSF"). The pegylated protein is also stabilized with regard to salts. The beneficial effects of pegylation on stabilizing enzymes in organic solvents has also been reported, see Inada, Y., et al; Tibtech 190-194 (1986). This latter point may have practical implications in the tablet formulation of the GSCF molecules.

G-CSF and analogs thereof have also reportedly been modified. EP O 473 268, "Continuous Release Pharmaceutical Compositions Comprising a Polypeptide Covalently Conjugated To A Water Soluble Polymer," reportedly describes the use of various G-CSF and derivatives covalently conjugated to a water soluble particle polymer, such as polyethylene glycol. Of course, with additional chemical moieties attached, the biologically active molecule is enlarged.

US Patent US-A-5 985 265 discloses N-terminally chemically modified protein compositions and methods, including modification of G-CSF and chemical modification of another protein, consensus interferon. As will be discussed in more detail below, chemically modified consensus interferon has demonstrated biological activity, such as anti-viral activity. An oral dosage formulation of chemically modified consensus interferon, the subject of another working example described below would also be desirable.

### SUMMARY OF THE INVENTION

The present invention is directed to the oral administration of a chemically modified protein, and delivery of the protein to the blood stream for therapeutic effect. Importantly, and surprisingly, it has been found that chemically modified biologically active proteins may survive in the intestine (with or without additional formulation), and pass through the lining of the intestine to the blood stream. Surprisingly, as demonstrated with pegylated G-CSF, not only did the protein survive, but it produced observable biological effects.

The examples below illustrate this. In a mammalian system, pegylated G-CSF is administered directly to the intestine. The animals tested uniformly exhibited higher total white blood cell counts than animals treated with non-pegylated G-CSF or vehicle. While the precise mechanisms are not defined, initial observations indicate that the chemical modification prevents proteolysis of the protein, and slows the clearance rate of the protein from the systemic circulation. The mechanism by which the lining of the intestine allows for uptake of the pegylated G-CSF into the blood stream, however, is not understood.

Therefore, one aspect of the present invention relates to compositions for the oral administration of a chemically modified G-CSF. Another aspect of the present invention relates to pegylated G-CSF in a pharmaceutically acceptable oral dosage formulation.

In general, G-CSF useful in the practice of this invention may be a form isolated from mammalian organisms or, alternatively, a product of chemical synthetic procedures or of prokaryotic or eukaryotic host expression of exogenous DNA sequences obtained by genomic or cDNA cloning or by DNA synthesis. Suitable prokaryotic hosts include various bacteria (e.g., E. coli); suitable eukaryotic hosts include yeast (e.g., S. cerevisiae) and mammalian cells (e.g., Chinese hamster ovary cells, monkey cells). Depending upon the host employed, the G-CSF expression product may be glycosylated with mammalian or other eukaryotic carbohydrates, or it may be non-glycosylated. The G-CSF expression product may also include an initial methionine amino acid residue (at position -1). The present invention contemplates the use of any and all such forms of G-CSF, although recombinant G-CSF, especially E. coli derived, is preferred, for, among other things, greatest commercial practicality.

Certain G-CSF analogs have been reported to be biologically functional, and these may also be chemically modified, by, for example, the addition of one or more polyethylene glycol molecules. Examples of G-CSF analogs which have been reported to have biological activity are those set forth in EP O 473 268 and EP O 272 423, although no representation is made with regard to the activity of each analog reportedly disclosed.

The chemical modification contemplated is the attachment of at least one moiety to the G-CSF molecule itself, where said moiety permits (a) inhibition of proteolysis; and (b) uptake into the blood stream from the intestine. Also desired is the increase in overall stability of the protein and increase in circulation time in the body. Examples of such moieties include: Polyethylene glycol, copolymers of ethylene glycol and propylene glycol, carboxymethyl cellulose, dextran, polyvinyl alcohol, polyvinyl pyrrolidone and polyproline. Abuchowski and Davis, Soluble Polymer-Enzyme Adducts. In: "Enzymes as Drugs", Hocenberg and Roberts, eds., Wiley-Interscience, New York, NY, (1981), pp 367-383; Newmark, et al., J. Appl. Biochem. 4: 185-189 (1982). Other polymers that could be used are poly-1,3-dioxolane and poly-1,3,6-tioxocane.

The preferred chemical moiety is polyethylene glycol. The preferred polyethylene glycol molecules are those which act to increase the half life of the protein in vivo, typically those PEG molecules with a molecular weight , of between about 6,000. The term "about" is used to reflect the approximate average molecular weight of a polyethylene glycol preparation, recognizing that some molecules in the preparation will weigh more, some less. The PEG used in the working examples described below had a molecular weight of about 6000.

The polyethylene glycol molecules (or other chemical moieties) should be attached to the protein with consideration of effects on functional or antigenic domains. The method for attachment of the polyethylene glycol molecules may vary, and there are a number of methods available to those skilled in the art. E.g., EP 0 401 384 (coupling PEG to G-CSF), see also Malik et al., Exp. Hematol. 20: 1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues glutamic acid residues and the C-terminal amino acid residue. Sulfhydrl groups may also be used as a reactive group for attaching the polyethylene glycol molecule(s). Preferred for therapeutic purposes is attachment at an amino group, such as attachment at the N-terminus or lysine group. Attachment at residues important for G-CSF receptor binding should be avoided. Attachment at residues found in external loops connecting alpha helices or the N-terminus is preferred. See, Osslund et al., PNAS-USA 90: 5167-5171 (1993) (describing the three dimensional conformation of recombinant human G-CSF).

The number of polyethylene glycol molecules so attached may vary, and one skilled in the art will be able to ascertain the effect on function. As noted in more detail below, the pegylated G-CSF preferred herein is predominantly tri-tetra pegylated with PEG 6000, i.e., a population of G-CSF molecule having three or four PEG 6000 molecules attached, with a minority of molecules having more or fewer polyethylene glycol molecules attached.

Contemplated for use herein are oral solid dosage forms, which are described generally in Remington's Pharmaceutical Sciences, 18th Ed.1990 (Mack Publishing Co. Easton PA 18042) at Chapter 89. Solid dosage forms include tablets, capsules, pills, troches or lozenges, cachets or pellets. Also, liposomal or proteinoid encapsulation may be used to formulate the present compositions (as, for example, proteinoid microspheres reported in U.S. Patent No. 4,925,673). Liposomal encapsulation may be used and the liposomes may be derivatized with various polymers (E.g., U.S. Patent No. 5,013,556). A description of possible soliddosage forms for the therapeutic is given by Marshall, K. In: *Modern Pharmaceutics* Edited by G.S. Banker and C.T. Rhodes Chapter 10, 1979.

In general, the formulation will include the chemically modified protein, and inert ingredients which allow for protection against the stomach environment, and release of the biologically active material in the intestine.

One preferred composition is PEG-G-CSF associated with an anionic lipid. As described more fully in Example 6 below, PEG-G-CSF associated with an anionic lipid demonstrated enhanced biological effects when delivered to the gut. Preferably, dioleoyl phosphatidylglycerol (DOPG) is used as an anionic lipid, but other anionic lipids may be used. The lipid vesicles useful in the compositions of the present invention are those negatively charged liposomes capable of interacting with PEG-C-CSF. Particular lipids contemplated for use include: dioleoylphosphatidylglycerol (DOPG), dimyristoylphosphatidylglycerol (DMPG), dipalmitoylphosphatidylglycerol (DPPG), egg phosphatidylglycerol, dioleoylphosphatidylethanolamine (DOPE), egg phosphatidylethanolamine, dioleoylphosphatidic acid (DOPA), dimyristoylphosphatidic acid (DMPA), dipalmitoylphosphatidic acid (DPPA), dioleoylphosphatidylserine (DOPS), dimyristoylphosphatidylserine (DMPS), dipalmitoylphosphatidylserine (DPPS), egg phosphatidylserine, lysophosphatidylglycerol, lysophosphatidylethanolamine, and lysophosphatidylserine. Depending on the particular lipid utilized, the amount of lipid could vary, and may be used in different combinations. Other materials and methods relating to use of anionic lipids are described in US Patent US-A-5 874 075 co-owned entitled, Stable Proteins: Phospholipid Compositions and Methods, herein incorporated by reference, and Collins et al., entitled Enhanced stability of granulocyte colony stimulating factor (G-CSF) after insertion into lipid membranes, J. Biochem. (under review).

The preferred location of release is the duodenum, as will be demonstrated below. Although duodenal release is preferable for optimal biological effect for a given dose, release throughout the gut results in uptake of the PEG-G-CSF as demonstrated below. One skilled in the art has available formulations which will not dissolve in the stomach, yet will release the material in the duodenum or elsewhere in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit® L30D, Aquateric®, cellulose acetate phthalate (CAP), Eudragit® L, Eudragit® S, and Shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic i.e. powder; for liquid forms, a soft gelatin shell may be used. The shell material of cachets could be thick starch or other edible paper. For pills, lozenges, molded tablets or tablet triturates, moist massing techniques can be used.

The therapeutic can be included in the formulation as fine multiparticulates in the form of granules or pellets of particle size about lmm. The formulation of the material for capsule administration could also be as a powder, lightly compressed plugs or even as tablets. The therapeutic could be prepared by compression.

Colorants and flavoring agents may all be included.

One may dilute or increase the volume of the therapeutic with an inert material. These diluents could include carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo®, Emdex®, STA-Rx® 1500, Emcompress® and Avicell®.

Disintegrants may be included in the formulation of the therapeutic into a solid dosage form. Materials used as disintegrates include but are not limited to starch including the commercial disintegrant based on starch, Explotab®. Sodium starch glycolate, Amberlite®, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An antifrictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential nonionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the PEG-G-CSF either alone or as a mixture in different ratios.

Additives which potentially enhance uptake of the cytokine are for instance the fatty acids oleic acid, linoleic acid and linolenic acid.

Controlled release formulation may be desirable. The drug could be incorporated into an inert matrix which permits release by either diffusion or leaching mechanisms i.e. gums. Slowly degenerating matrices may also be incorporated into the formulation. Another form of a controlled release of this therapeutic is by a method based on the Oros therapeutic system (Alza Corp.), i.e. the drug is enclosed in a semipermeable membrane which allows water to enter and push drug out through a single small opening due to osmotic effects. Some entric coatings also have a delayed release effect.

Other coatings may be used for the formulation. These include a variety of sugars which could be applied in a coating pan. The therapeutic agent could also be given in a film coated tablet and the materials used in this instance are divided into 2 groups. The first are the nonenteric materials and include methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, providone and the polyethylene glycols. The second group consists of the enteric materials already described that are commonly esters of phthalic acid.

A mix of materials might be used to provide the optimum film coating. Film coating may be carried out in a pan coater or in a fluidized bed or by compression coating.

The preferred formulation for oral delivery of G-CSF is recombinant human G-CSF (produced in a bacterial host for commercial practicability), such as Neupogen®, available from Amgen Inc., Thousand Oaks, California 91320-1789, di-tri-tetra pegylated as described in more detail below, and formulated so as to deliver the pegylated G-CSF to the small intestine. As will be demonstrated below, the small intestine, more particularly, the duodenum is the preferred location for release of the pegylated G-CSF from inert materials.

Also contemplated herein are processes for preparing the above oral dosage forms, as well as methods of treating a mammal in need thereof by orally administering an oral formulation of chemically modified protein. Preferred is a process for preparing an oral dosage formulation of G-CSF comprised of: (a) chemically modifying said G-CSF; and, (b) formulating such chemically modified G-CSF with a pharmaceutically acceptable carrier for oral administration.

Another aspect of the present invention includes the use of the chemically modified G-CSF for the manufacture of a medicament useful for treating a mammal for a condition characterized by a decrease in hematopoietic function comprised of the oral administration of chemically modified G-CSF, which may include a pharmaceutically acceptable oral formulation.

Formulations specific for certain indications may include other agents which are not inert, such as antibiotics, such as ceftriaxone, for the concomitant treatment of infection. Other non-inert agents include chemotherapy agents.

Conditions alleviated or modulated by the oral administration of chemically modified G-CSF (or analogs) are typically those characterized by a reduced hematopoietic or immune function, and, more specifically, a reduced neutrophil count. Such conditions may be induced as a course of therapy for other purposes, such as chemotherapy or radiation therapy. Such conditions may result from infectious disease, such as bacterial, viral, fungal or other infectious disease. For example, sepsis results from bacterial infection. Or, such condition may be hereditary or environmentally caused, such as severe chronic neutropenia or leukaemias. Age may also play a factor, as in the geriatric setting, patients may have a reduced neutrophil count or reduced neutrophil mobilization. Some of such conditions are reviewed in Filgrastim (r-met Hu G-CSF) in Clinical Practice, Morstyn, G. and T.M. Dexter, eds., Marcel Dekker, Inc., N.Y., N.Y. (1993), 351 pp. Other less-studied conditions which may be alleviated or modulated by oral administration may include the reduction of lipids (or cholesterol) in the blood stream, and certain cardiovascular conditions, as G-CSF may induce production of plasminogen activators. The mode of action of G-CSF (or analogs) in these settings is not well understood at present.

Administration may be in combination with other agents such as antibiotics, other hematopoietic factors, such as the interleukins (IL-1, IL-2, IL-3, IL-4, IL-5, IL-6, IL-7, IL-8, IL-9, IL-10, IL-11 and IL-12), early acting factors such as Stem Cell Factor or FLT3-L, erythropoietin, GM-CSF, IGF's (such as I and II), M-CSF, interferons (such as, but not limited to alpha, beta, gamma, and consensus), LIF, and CSF-1. Those skilled in the art will recognize when therapeutic effectiveness will require co-administration of a member of the group above, either simultaneously or in sequence. The co-administration may be via a different route (e.g., injection or infusion), or may be oral, nasal or pulmonary as a skilled practitioner will recognize.

As further studies are conducted, information will emerge regarding appropriate dosage levels for treatment of various conditions in various patients, and the ordinary skilled worker, considering the therapeutic context, age and general health of the recipient, will be able to ascertain proper dosing. Generally, dosage will be between 0.01 µg/kg body weight, (calculating the mass of the G-CSF alone, without chemical modification), and 100 µg/kg (based on the same).

The Examples below illustrate the working of the present invention.

Example 1 details the methods of preparing recombinant human G-CSF and pegylation thereof.

Example 2 describes an in vitro demonstration that a chemically modified protein (pegylated G-CSF) resists proteolysis by trypsin, which is found in the intestine.

Example 3 describes the in vivo model used to demonstrate the oral administration of a chemically modified protein. In rats, pegylated G-CSF was administered directly to the duodenum, either via an infusion pump or by bolus administration. The animals were allowed to recover, and blood was withdrawn at varying intervals to ascertain two parameters, total white blood cell count, and serum levels of G-CSF (via antibody detection). Intraduodenal bioequivalence as compared to intravenous injection was determined. The results demonstrate (1) the animals with pegylated G-CSF so administered demonstrated an increased white blood cell count over controls (with non-pegylated G-CSF or vehicle only); and (2) the animals with pegylated G-CSF administered demonstrated increased serum levels of G-CSF over controls (with non-pegylated G-CSF or vehicle only). This shows that the pegylated, biologically active G-CSF not only survived the conditions in the duodenum, but also permeated the intestinal lining to get into the blood stream at levels sufficient to stimulate a therapeutic response.

Example 4 presents additional data for serum levels of G-CSF using iodinated PEG-G-CSF, which provides for more sensitivity than antibody detection. Using the more sensitive assay, steady state serum levels of the protein are demonstrated over the period of intraduodenal infusion.

Example 5 describes an in vivo protocol for ascertaining the optimum location in the gut for release of the biologically active pegylated G-CSF. This information is instructive for determining the precise oral dosage formulation, which an ordinary skilled artisan may prepare for release in this target location. Generally, in a rat model, portions of the gut were physically isolated by surgically tying off and cutting the sections (at the duodenum, jejunum, ileum or colon). Pegylated G-CSF was administered into the isolated intestinal section, and blood samples were monitored for serum levels of rhG-CSF by ELISA. While there was detectable levels of the PEG-G-CSF in the serum from all portions of the gut, the results indicate that PEG-G-CSF administered to the duodenum and the ileum is optimal (highest serum levels).

Example 6 demonstrates that PEG-G-CSF associated with a lipid carrier enhances the therapeutic response elicited by PEG-G-CSF delivered to the duodenum. PEG-C-CSF was formulated using an anionic lipid, and delivered intraduodenally. The results show a higher white blood cell count as compared to PEG-G-CSF alone.

### BRIEF DESCRIPTION OF THE DRAWINGS

FIGURE 1 illustrates the rodent gastrointestinal tract, and diagrams the in vivo model of intraduodenal delivery used herein.

FIGURE 2 illustrates the resistance of pegylated G-CSF to trypsin proteolysis in an in vitro assay.

FIGURE 3 illustrates the total white blood cell response to PEG-G-CSF given by intraduodenal infusion, as compared to PEG-G-CSF administered by i.v., and non-pegylated rhG-CSF and vehicle administered by intraduodenal infusion.

FIGURE 4 illustrates the serum levels of rhG-CSF following administration of PEG-G-CSF intravenously and intraduodenally by infusion.

FIGURE 5 illustrates the total white blood cell response to PEG-G-CSF administered by intraduodenal and intravenous bolus and non-pegylated G-CSF given by intraduodenal bolus alone.

FIGURE 6 illustrates the serum rhG-CSF levels in response to intraduodenal and intravenous bolus administration of PEG-G-CSF. Also shown is the serum rhG-CSF level in response to intraduodenal bolus administration of non-pegylated rhG-CSF.

FIGURE 7 (a) illustrates a comparison of intravenous and intraduodenal pump infusion of ¹²⁵I-labelled PEG-G-CSF serum levels. FIGURE 7 (b) illustrates a comparison of AUC for each rat following intravenous and intraduodenal administration of ¹²⁵I-PEG-G-CSF.

Figures 8 (a) and (b) illustrate serum levels of rhG-CSF after PEG-G-CSF administration to different sections of the rat gut.

FIGURE 9 is a bar graph illustrating the net average AUC of serum levels of rhG-CSF after administration of PEG-G-CSF to different sections of the rat gut.

FIGURE 10 (a) is a graph illustrating the effect of DOPG on total WBC response to intraduodenal infusion of rhG-CSF. FIGURE 10 (b) is a graph illustrating this response using PEG-G-CSF.

FIGURE 11 is a graph illustrating the effect of DOPG on serum levels of PEG-G-CSF after intraduodenal pump infusion.

### DETAILED DESCRIPTION OF THE INVENTION

### Example 1 : Preparation of Pegylated G-CSF

### A. Preparation of Recombinant Human met-G-CSF

Recombinant human met-G-CSF was prepared as described above according to methods in the Souza patent, U.S. Pat. No., 4,810,643. The rhG-CSF employed was an E. coli derived recombinant expression product having the amino acid sequence (encoded by the DNA sequence) shown below (Seq.ID NOs.1 and 2): (This was also the non-pegylated composition used for the control animals.) Alternatively one may use purchased Neupogen® for the following pegylation procedures (the U.S. package insert for which is herein incorporated by reference). Recombinant human material was used for the rodent studies herein. Of course, if one so desires when treating non-human mammals, one may use recombinant non-human G-CSF's, such as recombinant murine, bovine, canine, etc. See PCT WO 9105798 and PCT WO 8910932, for example.

### B. Preparation of Chemically Modified G-CSF

Recombinant human met-G-CSF with predominantly two, three or four polyethylene glycol molecules attached was used in the examples using pegylated G-CSF. Attachment was accomplished via the reactive amino groups. The mean molecular weight of the pegylated G-CSF was between about 36,500 Daltons and about 42,500 Daltons, with the molecular weight of the polyethylene glycol chains being about 6000 Daltons each. (The mean molecular weight for this material was between about 29kDa and about 90kDa, as determined by SDS PAGE.) As indicated above, the polyethylene glycol molecule employed may be of various sizes, however, previous studies (data not shown) indicated that using G-CSF pegylated with predominantly two to three molecules of PEG-2000 resulted in rapid clearance, and therefore, no sustained circulation (which may be undesirable for oral delivery). The level of polyethylene glycol derivatization was determined to be: monopegylated, 3.4%; dipegylated, 31.9%; tripegylated, 49.3% and tetrapegylated, 15.4%. The in vitro biological activity (as determined by H³thymidine uptake assays) was determine to be 9% as compared to non-pegylated recombinant met G-CSF. The in vivo biological activity was determined to be 268% of non-pegylated recombinant met G-CSF.

The following method was used to prepare the peglyated G-CSF used in the studies described herein.

The polyethylene glycol was prepared in three steps: First, the synthesis of the ethyl ester of α-carboxymethyl ω-methoxypolyethylene glycol (CM-MPEG) was performed. 8.3 mmol of monomethoxypolyethylene glycol (MPEG) from Union Carbide, (MW. = 6,000) was dissolved in 300 ml of t-butanol at 50°C under nitrogen. 84 mmol of ethyl bromoacetate was then added and incubated again O/N at 50°C. After filtering through a sintered glass funnel and the addition of 200 ml of methylene chloride, the filtrate was concentrated 5-fold under vacuum. The ethyl ester of CM-MPEG was then precipitated by addition of 1 volume of the concentrated filtrate to 5-10 volumes of diethyl ether at 4°C, and collected on a sintered glass funnel and dried.

Next, the synthesis of α-carboxymethyl ω-methoxypolyethylene glycol (CM-MPEG) was performed. 50 g of the CM-MPEG ethyl ester was dissolved in 200 ml of 0.1 M NaOH. After incubation O/N at room temperature under nitrogen, the solution was cooled to 4°C and the pH adjusted to 3 with 2 N HCl. NaCl was added to saturation before extraction (3x) with equal volumes of methylene chloride. The combined organic phase was dried over anhydrous magnesium sulfate, filtered and concentrated to a final volume of 100 ml. The CM-MPEG was precipitated by addition to 500 ml of diethyl ether at 4°C, collected, and 50 g was redissolved in 150 ml of 0.1 M NaOH, the CM-MPEG was again precipitated by addition to 500 ml of diethyl ether at 4°C, collected and dried.

Next, the synthesis of N-hydroxysuccinimidyl ester of carboxymethyl methoxypolyethylene glycol (SCM-MPEG) was completed. In 120 ml of anhydrous methylene chloride was combined 5 mmol of the CM-MPEG, 10 mmol of N-hydroxy succinimide (NHS) and 10 mmol of dicyclohexycarbodiimide (DCC). After incubation for 8 hours at room temperature, the precipitated dicyclohexylurea was removed by filtration and the filtrate concentrated to 50 ml prior to addition to 600 ml of diethyl ether at 4°C.

The precipitated SCM-MPEG was collected by filtration on a sintered glass funnel and redissolved in anhydrous methylene chloride. After a second precipitation in diethyl ether, the SCM-MPEG was collected and dried. The SCM-MPEG was characterized by spectroscopic analysis and HPLC prior to conjugation to rhG-CSF.

To a 100 ml solution of rhG-CSF 10 mg/ml, in 100 mM Bicine pH 8.0, was added a 15 fold molar excess of the N-hydroxysuccinimidyl ester of carboxymethyl methoxypolyethylene glycol (SCM-MPEG, prepared as above). The reaction was for 1 hour at room temperature prior to dilution (x5) with distilled water to a total volume of 500 ml. The pH was adjusted to 4.0 with 1mM HCl.

The PEG-G-CSF was purified by FPLC using a Toyopearl SP 550C column (5 x 17 cm)(Pharmacia), prewashed with 700 ml of 0.2N NaOH, and pre-equilibrated with 1.3 L of column buffer, 20mM sodium acetate buffer pH4.0. The reaction mixture was loaded onto the column at a flow rate of 8 ml/minute, and the column was then washed with 1 L of the column buffer. 1.3 L of eluting buffer, column buffer containing 1 M NaCl, was pumped onto the column in a step gradient, and the PEG-G-CSF was eluted at 350 mM NaCl.

The fractions containing the PEG-G-CSF were pooled, concentrated to approximately 100 ml in an Amicon stirred cell using a YM10, 76 mm diameter Diaflo ultrafiltration membrane (Amicon). The PEG-G-CSF was then buffer exchanged using 600 ml of formulation buffer, 10 mM sodium acetate pH 4.0 and 5% mannitol and 0.004% Tween 80. The A₂₈₀ was determined and the protein diluted to 1mg/ml with formulation buffer, filter sterilized, and vialed.

The in vitro biological activity of the pegylated G-CSF was determined by measuring the stimulated uptake of ³H thymidine into mouse bone marrow cells prior to use in the studies below. The in vivo biological activity was also determined prior to use, by subcutaneous injection of hamsters (with 20 or 100 µg/kg PEG-G-CSF) and measuring total white blood cell count. Bioactivity as compared to non-pegylated G-CSF was calculated as the area under the WBC/time curve after subtracting the vehicle control curve. Relative bioactivity of the PEG-G-CSF was expressed as the percentage bioactivity compared to unmodified G-CSF (AUCₜₑₛₜ/AUC_{G-CSF} x 100).

### Example 2: In Vitro Protection From Preteases Found In The Intestine

This study demonstrates that in vitro, pegylated G-CSF is extremely resistant (without other protective formulation) to proteolysis by the enzyme trypsin which is found in the intestine. While not conclusive, this model is indicative of in vivo conditions in the intestine because roughly the same proportions of enzymes, and physiological conditions (pH, temperature, salinity) were used.

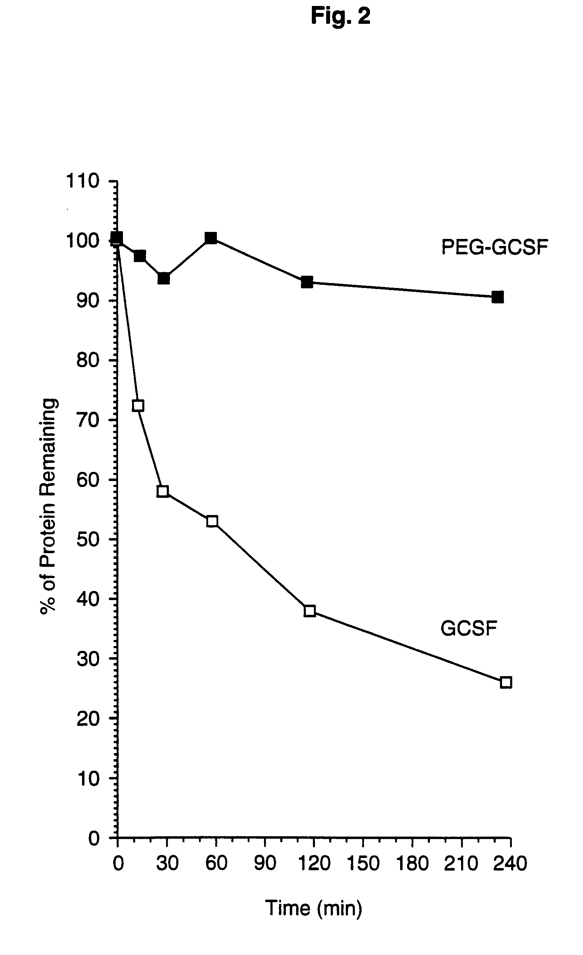

Generally, pegylated G-CSF (prepared as above) was incubated with trypsin, and the reaction was stopped at various time intervals over a 4 hour incubation. Samples taken at these times were tested for the amount of degradation by SDS-PAGE and Western blotting using antibodies against G-CSF, detected using iodinated protein A. The results, as presented in the graph at FIGURE 2 demonstrate the protective effects of pegylation: after 30 minutes, greater than 90% of the pegylated material was intact, whereas approximately 55% of the non-pegylated material was intact; after 240 minutes, at least 90% of the pegylated material remained while the non-pegylated material dropped to less than 30%. In vivo, there would be other enzymes, and additional factors affecting the rate of degradation.

The methods were as follows: rhG-CSF or PEG-GCSF as prepared above, at 100 µg/ml, in a total volume of 5 ml of phosphate buffered saline, (PBS) was incubated at 37°C with trypsin (1 µg/ml, Sigma St. Louis, MO). For the times indicated at 37°C. At the appropriate time points, 200 µl of sample was withdrawn and added to an Eppendorf tube at 4°C containing 9 µl of a protease inhibitor cocktail, consisting of N-tosyl-L-lysine chloromethyl ketone (TLCK), 20 µg; (4-amidinophenyl) methanesulfonyl fluoride (APMSF), 16 µg; and alpha 2-macroglobulin, 1IU, (all from Boehringer Mannheim, Indianapolis, IN). After thorough mixing, 5 µl of the sample (5 µg of G-CSF) was diluted to 5 µg /ml in PBS. 50 ng of the protein were then run under reducing conditions as described by Laemmli (Nature 227: 680-685 (1970)) on SDS-PAGE (Integrated Separations Systems or ISS, Natich, MA). After transfer, the protein was detected by incubation with a polyclonal antibody to rhG-CSF. The bound anti-G-CSF antibody was then detected by incubation of the blot with ¹²⁵I-protein A (Amersham, Arlington Heights, IL) and autoradiography. Quantitation of the remaining intact protein and of the degradation products was by cutting and counting of the Immobilon using the autoradiograph as the template.

### Example 3: In Vivo Duodenal Administration of Pegylated G-CSF Results In Biological Effects

The in vivo rat model, in which PEG-G-CSF is administered directly to the duodenum, is indicative of oral administration because, as pointed out above, formulations exist for delivering therapeutics to the intestine, beyond the hostile environment of the mouth, esophagus and stomach. The animals with pegylated G-CSF so administered demonstrated an increased white blood cell count over controls (with vehicle only). This shows that the pegylated, biologically active G-CSF not only survived the conditions in the duodenum, but also passed through the intestinal lining to the blood stream.

Further analysis compared the effects of intraduodenal administration to intravenous administration. This bioequivalence analysis demonstrated that as compared to intravenous administration, intraduodenally administered PEG-G-CSF (1) had 4-5% of the biological effectiveness (as ascertained by total white blood cell count after 90 hours), and (2) had approximately 2% of the serum level (as determined by ELISA after 90 hours). The mode of dosing was also compared i.e. chronic administration vs. acute, by a comparison of responses to infused and bolus administered PEG-GCSF.

### Materials and Methods

A. Animals. Male SPF Sprague-Dawley rats, weighing between 250-350 grams, treated in accordance with all applicable laws and regulations, were used. For each cohort below, either four or five animals were used.

B. Surgery. Animals were anesthetized with 50mg/kg of intraperitoneal Nembutol. The duodenum in each animal was exposed, and a small incision was made in the wall of the duodenum. A catheter (used for the delivery of the drug) [10 cm silastic medical grade tubing, 0.02 x 0.037 in., Baxter, Irvine, CA] was inserted to the distal end of the duodenum (approximately 8 cm) so that PEG-G-CSF would not enter the blood stream through the surgical incision (thereby having an artifactual effect). Moreover, release of the drug at the distal end of the duodenum (that part proximate to the jejunum) provides some indication of the effect of a formulation designed to release active compound into the duodenum (i.e., the typical release might be just above the duodenum/jejunum border). Release at the distal end avoids bile influx which contains proteases. After administration of PEG-G-CSF, the incision was closed with a purse string suture, and the animals were maintained as usual.

C. Administration. Administration of the pegylated G-CSF was accomplished in two ways, (1) via direct bolus administration through the catheter, and (2) via implanted pump infusion (for continuous administration over a 24 hour period). For each type of administration, a non-pegylated G-CSF control group was used, as were vehicle controls.

For intraduodenal bolus administration, PEG-G-CSF (as prepared above) was placed in a 1 cc syringe with a tubing adaptor, and then injected directly into the duodenum through the catheter. The proteins at the indicated doses were injected into the duodenum in 200 µl of formulation buffer, 10 mM sodium acetate pH 4.0 and .004% Tween 80. The catheter was withdrawn, and the suture closed tightly. The animal was allowed to recover.

For intravenous bolus administration (used as controls) 200 µl of formulation buffer containing the required dose of protein was administered through the penile vein.

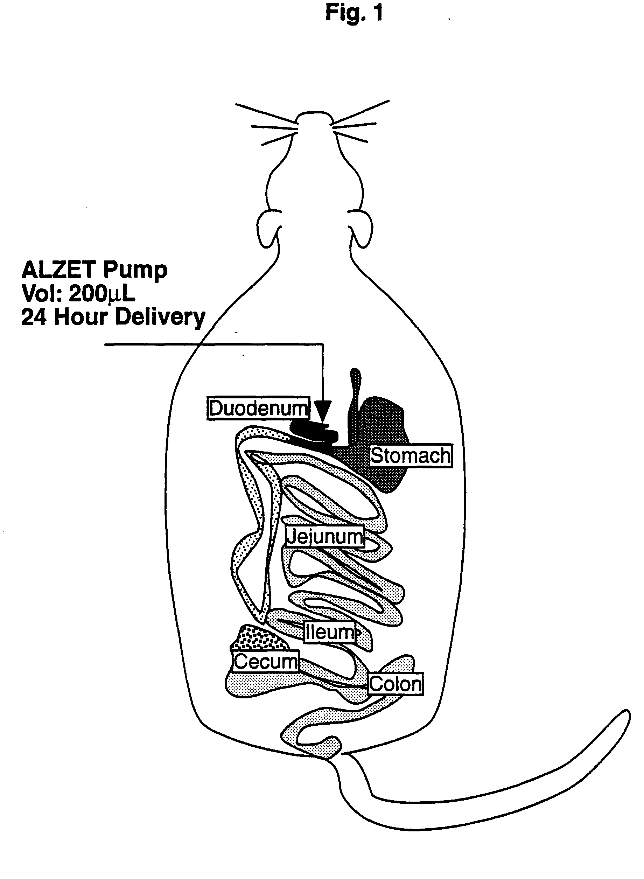

For the intraduodenal pump infusion, an osmotic pump [Alzet, mini-osmotic pump, model 2001D (Alza) Palo Alto, CA] was placed on the tip of the catheter located in the peritoneal cavity (See FIGURE 1). Prior to such placement, the pump was prefilled with pegylated G-CSF (as prepared above) or controls, at indicated dose, in 221 µl of formulation buffer, and the pump was activated via osmotic means (absorbing water from the animal to push the drug out) to deliver 8-9 µl/hr for 24 hours. In all cases, the value given for the dose refers to total dose over 24 hours. The incision was closed, and the animal was allowed to recover.

For the intravenous pump infusion of the proteins, an incision (approx. 3-4 cm ) was made under the neck of the rat. The left jugular vein was exposed, and the 10 cm silastic catheter was introduced 2 cm into the vein. The Alzet pump containing the proteins was attached to the catheter, and implanted into the nape of the neck between the shoulder blades.

D. Dosing. For intraduodenal infusion the animals were administered the proteins, both PEG-GCSF and non-pegylated GCSF at doses greater than 750 µg/kg over 24 hours (for actual amounts see Figures). For the intravenous infusion the doses of the proteins were less than 50 µg/kg over 24 hours. Animals receiving the proteins via intraduodenal bolus administration were given doses of 500 µg/kg whereas the intravenous bolus dosing was ~5 µg/kg.

E. Monitoring. For the intraduodenal infusion studies, blood samples (500 µl) were drawn from the tail vein of each of the test and control groups at twelve-hour intervals for 96 hours. For the bolus injection studies either intraduodenal or intravenous blood samples (500 µl) were drawn through an indwelling cannula in the right Jugular vein. The cannulas were implanted 2 days prior to drug administration to allow the animals to recover, and were kept patent by flushing twice daily with 100 µl of saline containing 20 U/ml of heparin.

Total white blood cell counts were determined using a Sysmex (Baxter, Irvine, CA) F-800 microcell counter. Serum was prepared by centrifuging the blood samples in an Eppendorf centrifuge at 12000 rpm, 11750 x g, for 15 minutes. The serum was removed and stored at -80°C until an ELISA for rhG-CSF could be performed.

Serum levels of PEG-G-CSF and non-pegylated G-CSF were determined by ELISA, containing a monoclonal antibody specific for G-CSF, (Quantikine, available from R&D Systems, Indianapolis, Indiana, US), according to the instructions, which are herein incorporated by reference. The standard curves were set up from 5000 pg/ml to 78 pg/ml of the exact same protein that had been administered to the animals. The serum levels of the proteins were then determined from the relevant curve.

### Results

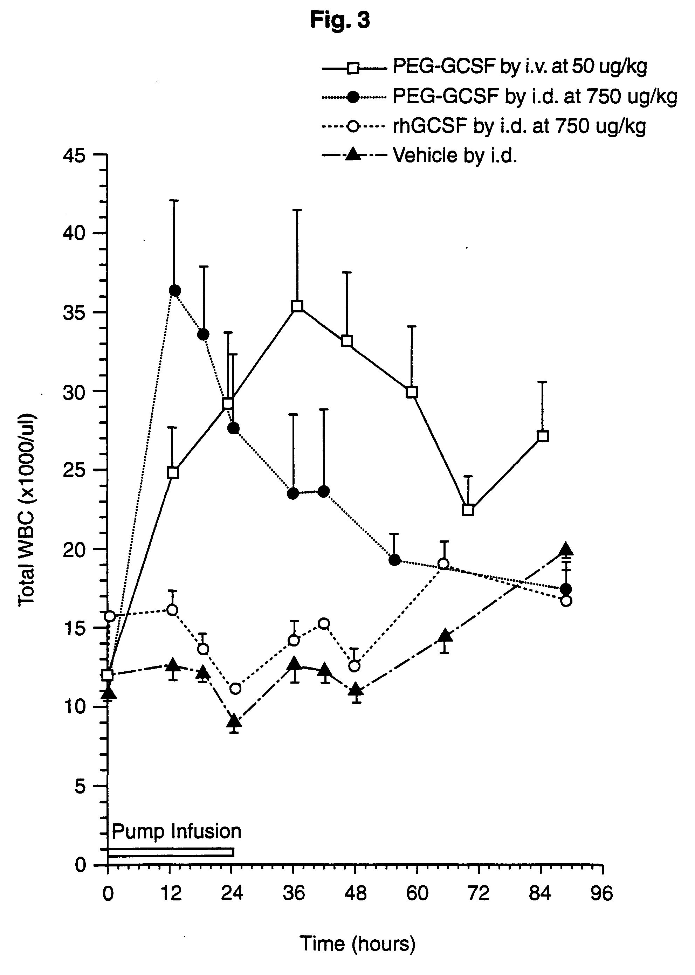

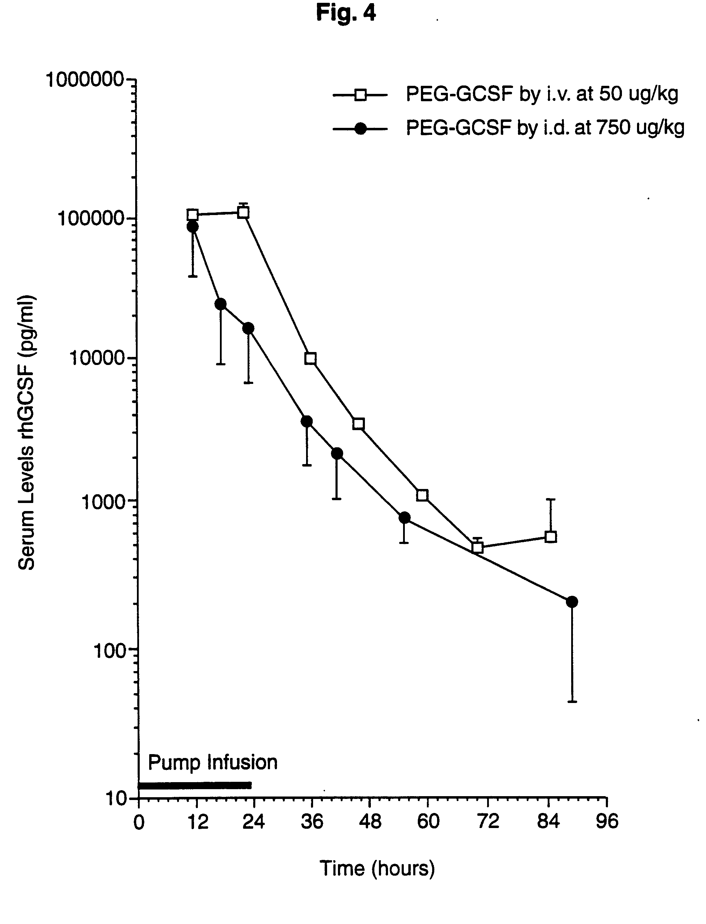

### 1. Intraduodenal infusion (FIGURES 3 and 4).

As can be seen in Figure 3, the cohort receiving PEG-GCSF intraduodenally had much higher total white blood cell counts at 12 hours (~36,000/µl) than did the intraduodenal non-pegylated controls (~16,000/µl). One can also see that the latter group is not raised over the baseline (T = 0) WBC count, as is also the case for the i.d. vehicle group. (see FIGURE 3) An interesting point to note is that PEG-G-CSF given intraduodenally stimulates an earlier increase in white blood cells than intravenous administration. The doses, however, for the intraduodenal and intravenous administrations are very different (since the comparison of these responses was for the determination of bioequivalence, see Table 2). This earlier WBC increase may be a result of the different doses or routes of administration in that (a) there may be a difference in the rate of white blood cell production or (b) there may be a difference in the activation of neutrophils and therefore margination, or (c) a combination of both. Another observation is that neither the non-pegylated G-CSF nor vehicle cohorts showed elevated white blood cell counts until after 48 hours (after the PEG-G-CSF response began to decrease), and this may be due to rejection of the osmotic pump or other immune artifact.

The serum levels for the same experiment are shown in Figure 4. No values are shown for the non-pegylated G-CSF control group, since the ELISA assay showed no detectable serum levels of rhG-CSF (i.e. less than 50 pg/ml). The serum levels achieved by intraduodenal and intravenous infusion of PEG-G-CSF are not directly comparable due to the difference in dose. Instead these data were used for the determination of bioavailability and are shown in Table 2. One can see however that serum levels of PEG-G-CSF are highly elevated for the protein after intraduodenal infusion as compared to the undetectable levels after non-pegylated GCSF administration via the same route.

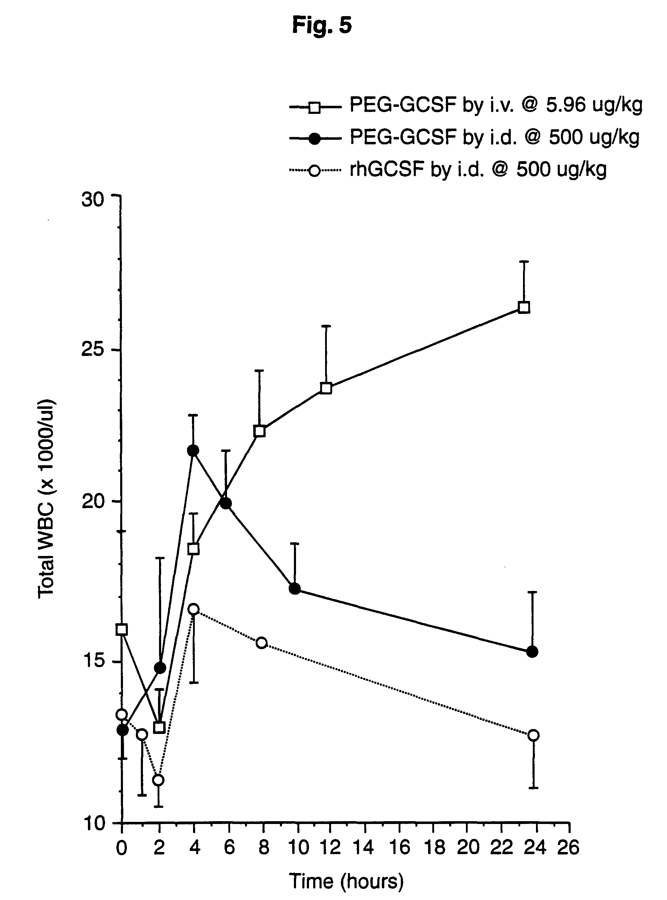

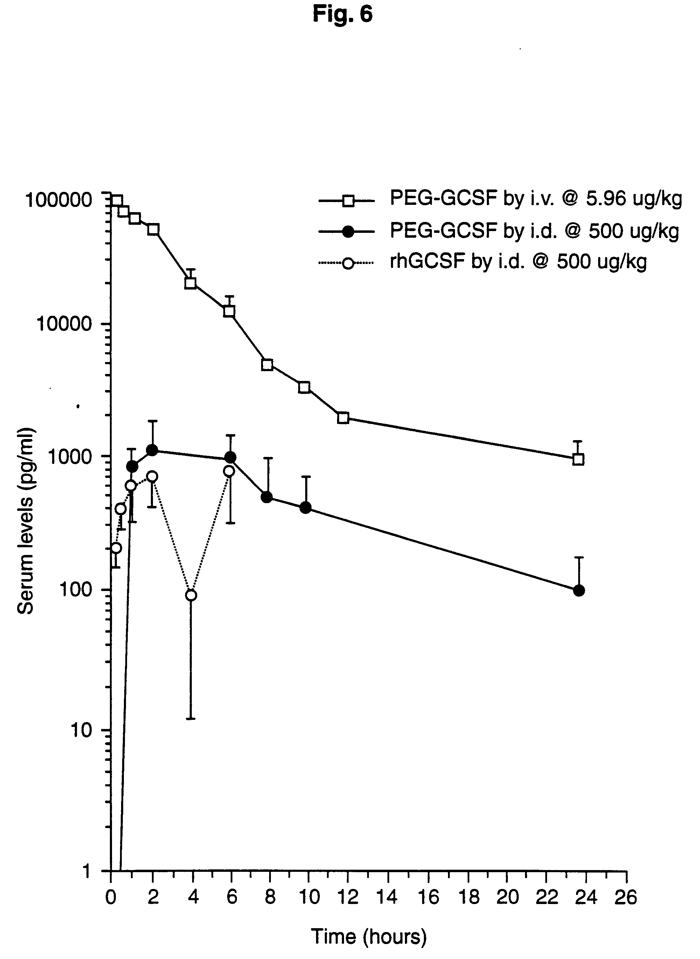

### 2. Bolus administration (FIGURES 5 and 6).

As can be seen in FIGURE 5, the total WBC for the test group at 5 hours was approximately 21,500/µl, whereas for the G-CSF control group, the level at 5 hours was much less (approximately 16000/µl) which was not significantly raised over baseline (T = 0). As can also be seen from this FIGURE, G-CSF alone produced some effect in the short term, indicating that the intestinal lining permitted traversal by both the larger pegylated and smaller non-altered molecules. The sustained WBC levels for the pegylated product indicate that there is protection from the duodenal environment, as well as increased serum circulation time as compared to non-pegylated GCSF. The same rapid increase in WBC is seen with the i.d. administration compared to i.v. FIGURE 6 illustrates the serum levels as determined by ELISA, of PEG-G-CSF administered by both the i.d. and i.v. routes, and non-pegylated material administered by the i.d. route. For the pegylated cohort, the serum levels remained relatively constant for the first six hours, and gradually decreased thereafter, the decrease parallelling that of the i.v. administered material. As can be seen, the serum levels for the non-pegylated G-CSF were half the values of the PEG-G-CSF group and were extremely variable (some animals had undetectable amounts) and below the level of detection in the entire group after 6 hours.

3. Bioequivalence analysis. An analysis was performed to compare intraduodenal administration of the proteins to intravenous administration. The results show that intraduodenal administration by the infusion method has between 4% and 5% of the biological effectiveness ("bioequivalence") of intravenously administered pegylated G-CSF, as determined by white blood cell count. These WBC count data are presented in Table 1, below. Bioavailability as determined by serum levels (1.8%) is somewhat lower than that determined from WBC (4.6%). The serum level data are presented in Table 2 below.

In general, % bioequivalence is determined by measuring the area under the white blood cell count curve ("AUC") for intraduodenally administered ("id") material (corrected for the vehicle), and dividing that number by the AUC for intravenously ("iv") administered material (again corrected for the vehicle). This number is multiplied by the reciprocal dosage. The product is multiplied by 100 for the percentage. For bioavailability in terms of serum, the calculation is the same.

The equation may be represented as:$\text{% Bioequivalence =} \frac{{\text{AUC}}_{\text{id}}}{{\text{AUC}}_{\text{iv}}} \text{X} \frac{{\text{Dose}}_{\text{iv}}}{{\text{Dose}}_{\text{id}}} \text{X 100}$

In the Tables below, the notation "ND" means not detectable.

**Table 1**

| Bioequivalence of PEG-G-CSFid vs. PEG-G-CSFᵢᵥ As Determined Using White Blood Cell Counts | | | | |
|---|---|---|---|---|
| Protein | Administration | Dose (µg/ kg) | Net Ave AUC (hours/AUC) | % Bio-equiv. |
| rhG-CSF | 24 hour infusion iv | 25 | 90hrs/1488 | 100 |
| rhG-CSF | 24 hour infusion id | 755 | 90hrs/ND | 0 |
| PEG-G-CSF | 24 hour infusion iv | 50 | 90hrs/1136 | 100 |
| PEG-G-CSF | 24 hour infusion id | 823 | 90hrs/852.24 | 4.6 |
| | | | | |
| rhG-CSF | bolus iv | 50 | 24hrs/216 | 100 |
| rhG-CSF | bolus id | 500 | 24hrs/40.2 | 1.86 |
| PEG-G-CSF | bolus iv | 5.96 | 24hrs/234 | 100 |
| PEG-G-CSF | bolus id | 500 | 24hrs/156 | 0.84 |

**Table 2**

| Bioavailability of PEG-G-CSFid vs. PEG-G-CSFᵢᵥ As Determined Using Serum Levels | | | | |
|---|---|---|---|---|
| Protein | Administration | Dose (µg/kg) | Net Ave.AUC (hours/AUC) | % Bio-avail. |
| rhG-CSF | 24 hour infusion iv | 25 | 90hrs/2.0x10⁵ | 100 |
| rhG-CSF | 24 hour infusion id | 755 | 90hrs/ND | 0 |
| PEG-G-CSF | 24 hour infusion iv | 50 | 90hrs/2.17x10⁶ | 100 |
| PEG-G-CSF | 24 hour infusion id | 823 | 90hrs/6.3x10⁵ | 1.8 |
| | | | | |
| rhG-CSF | bolus iv | 50 | 24hrs/7.23x10⁷ | 100 |
| rhG-CSF | bolus id | 500 | 24hrs/1.8x10³ | 0.00025 |
| PEG-G-CSF | bolus iv | 5.96 | 24hrs/2.7x10⁵ | 100 |
| PEG-G-CSF | bolus id | 500 | 24hrs/1.1x10⁴ | 0.05 |

Thus, importantly, Table 1 shows that after a 24 hour id infusion of PEG-G-CSF, material has entered the bloodstream and has a measurable biological response, which is much greater (4.6%) than that for native rhG-CSF (0%). In fact, non-pegylated rhG-CSF does not stimulate any white blood cell response when administered by infusion i.d., nor are there detectable levels of the protein in the serum.

In contrast, bolus administration of PEG-G-CSF and rhG-CSF did not result in such large differences between the two proteins. The reason for the almost equivalent WBC responses for the PEG-G-CSF and for native G-CSF probably lies in the fact that the time points were not taken beyond 24 hours and therefore the major part of the PEG-G-CSF response i.e. prolonged elevated WBC, was not measured. A comparison of the serum levels of PEG-G-CSF and rhG-CSF over just the 24 hour period shows much greater bioavailability of the pegylated protein, the AUC is 10-fold greater. One can see, however, that the serum levels following the bolus administration of PEG-G-CSF are much smaller than following the infusion method, 0.05% bioavailability compared to 1.8%. It would seem that the infusion method of administering the protein produces the best bioavailability and therapeutic responses and that a tablet formulation producing a prolonged or sustained exposure of the gut to PEG-G-CSF would be preferable.

These data are further illustrated in FIGURE 3. As can be seen, PEG-G-CSF by intraduodenal administration has an earlier effect on white blood cell count than PEG-G-CSF administered intravenously. Also shown are the vehicle and non-pegylated G-CSF controls, which show no such increase in white blood cell count. The increase shown at 48 hours for the vehicle may be due to rejection of the osmotic pump or other immune artifact.

FIGURE 4 further illustrates intravenous and intraduodenal administration of PEG-G-CSF. Although the doses administered are very different, FIGURE 4 shows that the clearance rate of the id administered PEG-GCSF is similar to that for intravenously administered material. Again, as shown by the data in Table 1, non-pegylated G-CSF serum levels were not measurable.

In summary, the in vivo studies here demonstrate the availability of a chemically modified protein for uptake by the intestine, and, importantly, the therapeutic activity of such protein. More particularly, the studies demonstrate that pegylated G-CSF delivered to the intestine is present in the blood stream and causes an increase in white blood cells, and that the oral formulation of such composition will be a useful therapeutic.

### Example 4: Confirmation of Serum Levels

One interesting observation using the ELISA assay was that, for the infusion system, the serum levels of PEG-GCSF dropped while the pump was in operation. In addition, the bioavailability of the protein given by the serum values was consistently lower than the bioequivalence values, i.e., the response, and this was especially true of the bolus administration data. To confirm these data, a more sensitive assay was used. The data were confirmed (see Table 3, below). One explanation for this occurrence is that the initial response to PEG-G-CSF causes a rapid rise in the neutrophil level. Creating this rapid rise also increased the apparent clearance of the protein, possibly due to an increase in the number of G-CSF receptors on the neutrophils. As the neutrophil count increases, the serum levels of the protein appear to decrease (because it is bound to the neutrophils and so does not appear in the serum and thus there is no accumulation of PEG-G-CSF in the serum). This is consistent with results published elsewhere. G. Morstyn et al., TIPS 10: 154-159 (1989); Layton et al., Blood 74: 1303-1307 (1989).

For this assay, ¹²⁵I-labelled PEG-G-CSF was used, as were iv and id methods as described above. The difference is the dosage, as here, 1/1000 of the dose was used as compared to the previous studies: 661 nanograms/kg for intravenous administration, and 728 nanograms/kg for intraduodenal administration (whereas microgram quantities were used previously). Total blood levels of TCA-precipitable ¹²⁵I label were determined in a Cobra 5000 gamma counter (Packard, Downers Grove IL), and the data converted to picograms per ml.

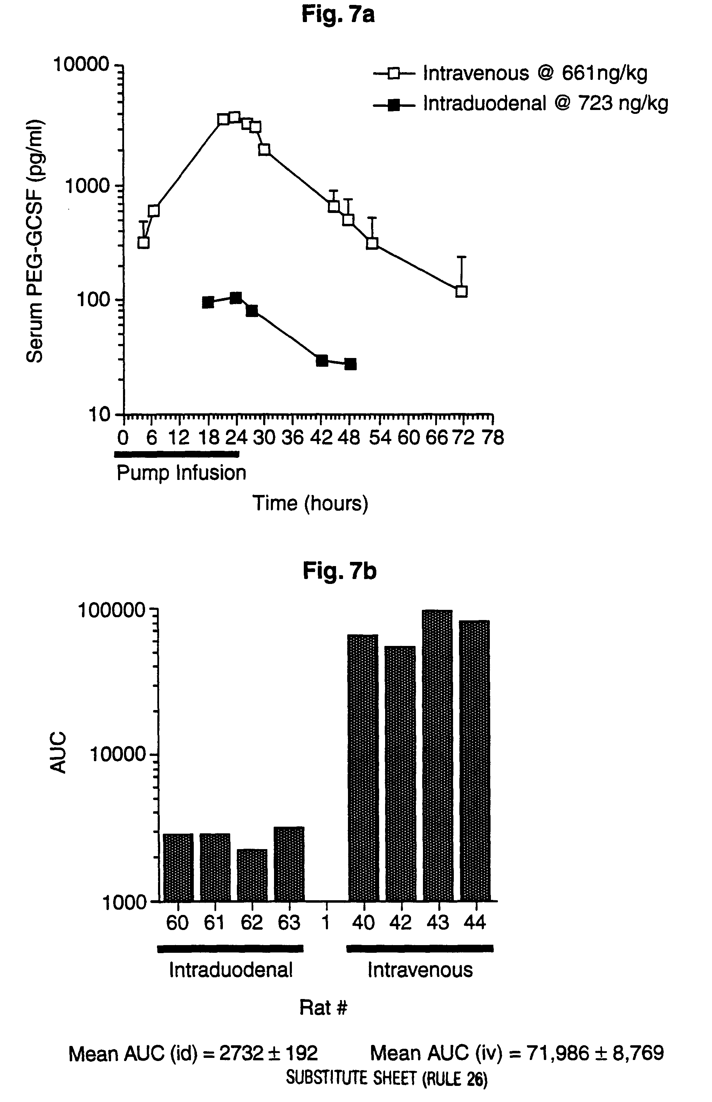

The results of both the intravenous and intraduodenal administration of the ¹²⁵I-labeled PEG-G-CSF are shown in FIGURE 7a. As one can see, by administering low, non-therapeutic doses of the proteins, and thus not stimulating neutrophil elevation, steady state levels of the PEG-G-CSF have been achieved by both routes. When the pumps have finished at 24 hours, levels of the protein drop in the blood in parallel as one would expect. Even with the increased sensitivity of detection of this method, blood levels are not detectable below 20 pg/ml (see id administration).

Calculation of the individual AUC for each animal in the cohort is shown in FIGURE 7b and without the change in clearance of the protein, is a more accurate measure of actual bioavailability. The data are summarized in Table 3 below.

**Table 3**

| Bioavailability of ¹²⁵I-labeled PEG-G-CSF_{id} vs. ¹²⁵I-labeled PEG-G-CSEᵢᵥ as determined using whole blood levels. | | | | |
|---|---|---|---|---|
| Protein | Administration | Doses (µg/kg) | Net Ave. AUC (hrs/AUC) | % Bioavailability |
| ¹²⁵I-PEG-G-CSF | 24 hour infusion i.v. | 0.661 | 48 hrs/ 71,986 ± 8769 | 100 |
| ¹²⁵I-PEG-G-CSF | 24 hour infusion i.d. | 0.728 | 48 hrs/ 2,732 ± 192 | 3.5 |

The data for the AUC give a value for the bioavailability of 3.5% as compared to intravenous administration, which is closer to the number for the bioequivalence given in Table 1 of 4.6%.

### Example 5: Localization of Delivery Target In the Small Intestine

As described above, a variety of oral dosage formulations are available in the art, and one aspect is formulation so that the tablet (or capsule, etc.) will dissolve in a desired location in the gut. This in situ study was designed to find the small intestine location yielding optimal (in this case, maximal) bioavailability as determined from serum levels of the protein. The results show that delivery to the duodenum and ileum produces the highest serum levels of the protein.

### Materials and Methods

The in situ closed loop animal model used here was a modified version of that described by Schilling and Mitra, Pharm. Res. 9: 1003-1009 (1992).

Animals. Male Sprague-Dawley rats weighing 200-250 g were fasted 16-20 hr prior to the experiment. Water was allowed ad libitum. The animals were anesthetized by an intraperitoneal injection of a mixture of 90 mg/kg ketamine and 10 mg/kg xylazine. One-third to one-half of the original dose was administered every 45-60 min thereafter to maintain anesthesia/analgesia. The core body temperature was maintained at 37°C by placing the animal on a heating pad.

IV Catheterization. Cannulation of the right external jugular vein was performed by inserting a 10 cm piece of Silastic tubing, (Baxter, Irvine, CA). A collar made from a 1 cm piece of PE 200 polyethylene tubing was attached to the outer end of the Silastic tubing. Before insertion, the cannula was filled with saline containing 10 U/ml heparin. A 23-gauge needle was inserted into the cannula and was used with a heparinized 1 ml syringe for the removal of blood samples.

Bile Duct Catheterization. Cannulation of the bile duct was necessary to prevent excess accumulation of bile in the non-ligated gut over the 4 hours of the experiment. A midline abdominal incision was made, and the duodenum and a small part of the intestine was pulled out and placed on a gauze pad moistened with physiological saline to expose the bile duct. Two ligatures were made, one ligature was tied tightly immediately in front of the pancreatic tissue to prevent the flow of bile, the second ligature was partly tied 5 mm from the first ligature and near to the liver. A polyethylene tube (0.28 mm id and 0.61 mm od) beveled at one end, was introduced into the bile duct, toward the liver, through a fine incision. The catheter was advanced past the second ligature which was then tightened to secure the catheter in the bile duct. The free ends of the first ligature were then secured.

ID Administration. Next, intestinal segments were measured with a string. Experiments were carried out in individual animals to test for PEG-G-CSF absorption from the duodenum (11 cm from the pylorus), the proximal jejunum (20 cm from the pylorus), the distal ileum (6 cm above the cecum), and the colon (10 cm from the cecum). The desired segment was opened at each end and a piece of Tygon tubing (4-mm o.d. from VWR Scientific, Cerritos, CA) was inserted into the proximal opening. A peristalic pump was employed to perfuse 30 ml of physiological saline (Abbott Laboratories, Chicago IL) at 37°C and 2 ml/min into the intestine to remove any residual gut contents. Each segment (10 cm) was ligated both above and below the incisions to prevent any fluid loss, and air was pumped through the segment to remove any residual saline. PEG-G-CSF solution in 500 µL of formulation buffer, 10 mM sodium acetate, pH 4.0, 5% mannitol and 0.004% Tween 80, at a dose of 750 µg/kg, was injected into the mid-portion of the segment using a 27 gauge half inch needle. The segment was carefully returned to its original position inside the peritoneal cavity and the abdominal cavity was closed with surgical staples. Blood samples (250 µL) were obtained at 0, 2, 5, 10, 15, 30, 60, 120, 180, and 240 minutes post administration for the determination of plasma rhG-CSF concentrations. Blood samples volumes throughout the experiment were replaced in the animal, with the same volume of physiological saline.

Intravenous Administration. To determine the bioavailability of enterally absorbed PEG G-CSF, the pegylated cytokine was administered via the penile vein (50 µg/kg in 100 uL of formulation buffer) of a fasted, iv and bile duct cannulated rat. Blood samples were obtained as per id administration.

Analysis. Plasma was separated by first collecting the blood into EDTA-coated Eppendorf tubes kept on ice, and then centrifuging at 10,000 rpm for 15 min. Serum samples were frozen and stored at -80°C until analysis for rhG-CSF by R&D Systems ELISA.

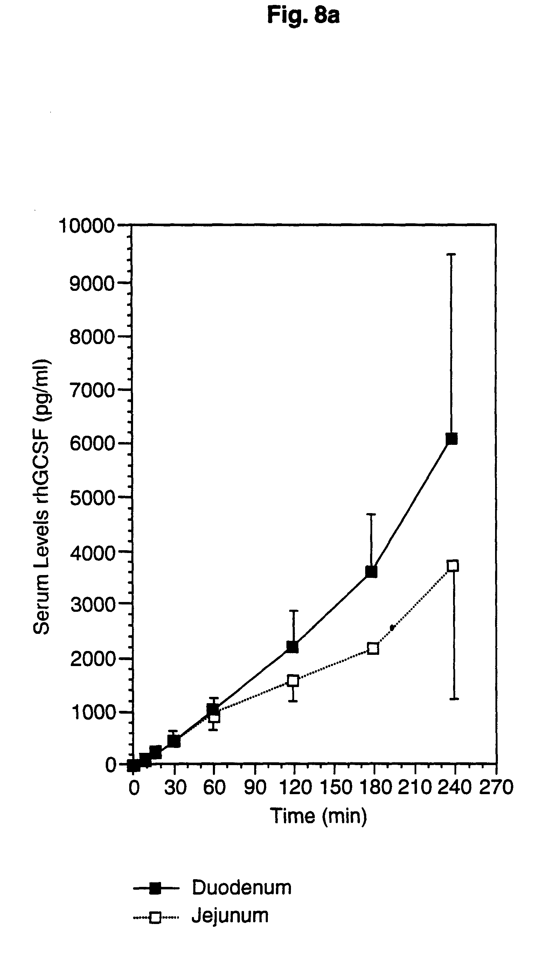

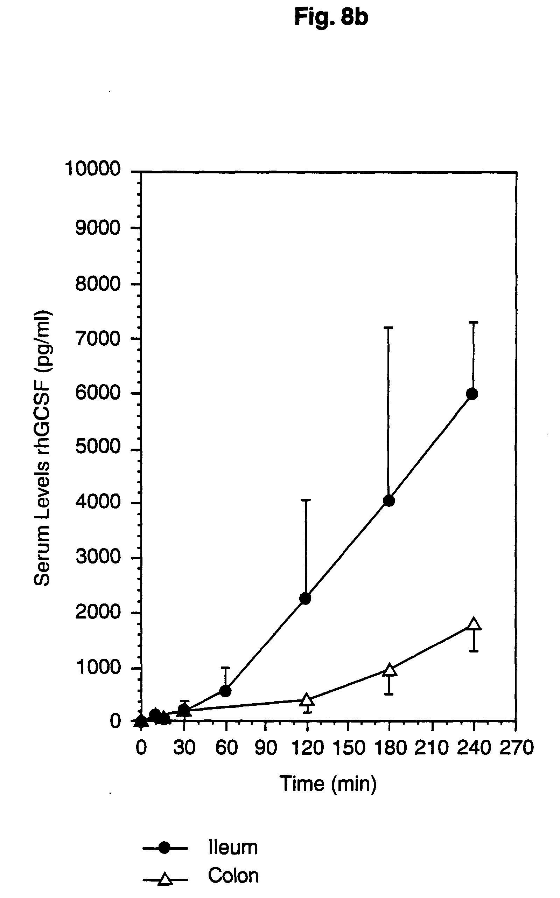

Results are presented in FIGURE 8. The data are the mean values from 3 separate experiments. The degree of error, as shown by error bars, may be due in part to the fact that the 3 animals for the group were studied on separate days. This would increase differences in each study, although corrections were made for certain changes, i.e. weight of the rats, etc. FIGURE 8 illustrates, however, that the higher regions of the gut i.e. duodenum and ileum, are preferable in terms of PEG-G-CSF absorption than the lower regions, such as the colon.

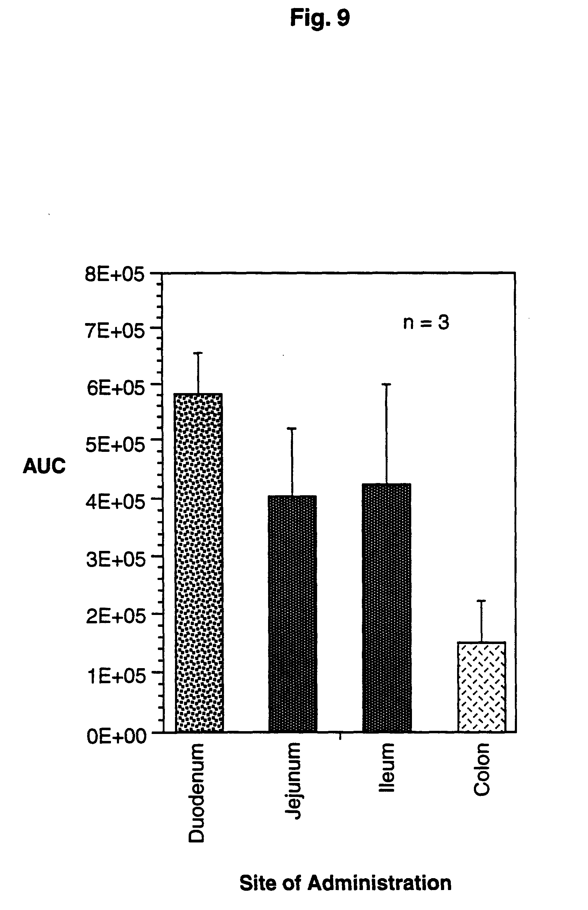

This fact is emphasized by the AUC analysis for the serum levels of the protein which are presented in FIGURE 9. Surprisingly, the data clearly show that the small intestine is the preferred site for an oral delivery formulation of PEG-G-CSF as opposed to the large intestine which is not preferable. The colon is generally thought to be the most leaky region of the gut and, apart from the bacterial flora present, less hostile to proteins than the more protease-active regions of the duodenum, jejunum and ileum.

Additional studies may provide more information regarding dosing and extrapolation of optimal formulation from species to species.

### Example 6: Formulation of PEG-G-CSF with Dioleoyl Phosphatidylglycerol

Recombinant human G-CSF is able to closely interact with a negatively charged lipid, which enhances stability of the G-CSF protein. PEG-G-CSF also forms this close interaction, with protective effects. This Example demonstrates that the protective effects have a positive impact on the intraduodenal bioavailability of PEG-GCSF after formulation of the protein with a negatively charged lipid.

The present example relates to the negatively charged lipid dioleoyl phosphatidylglycerol (DOPG). Other formulations using negatively charged lipids in association with proteins capable of forming the molten globular state are described in commonly owned, copending U.S.S.N. 08/132,413, "Stable Proteins: Phospholipid Composition and Methods" which is herein incorporated by reference. The use of such negatively charged lipids as binders in oral dosage formulations has been previously demonstrated, and may be useful for the oral dosages forms here described.

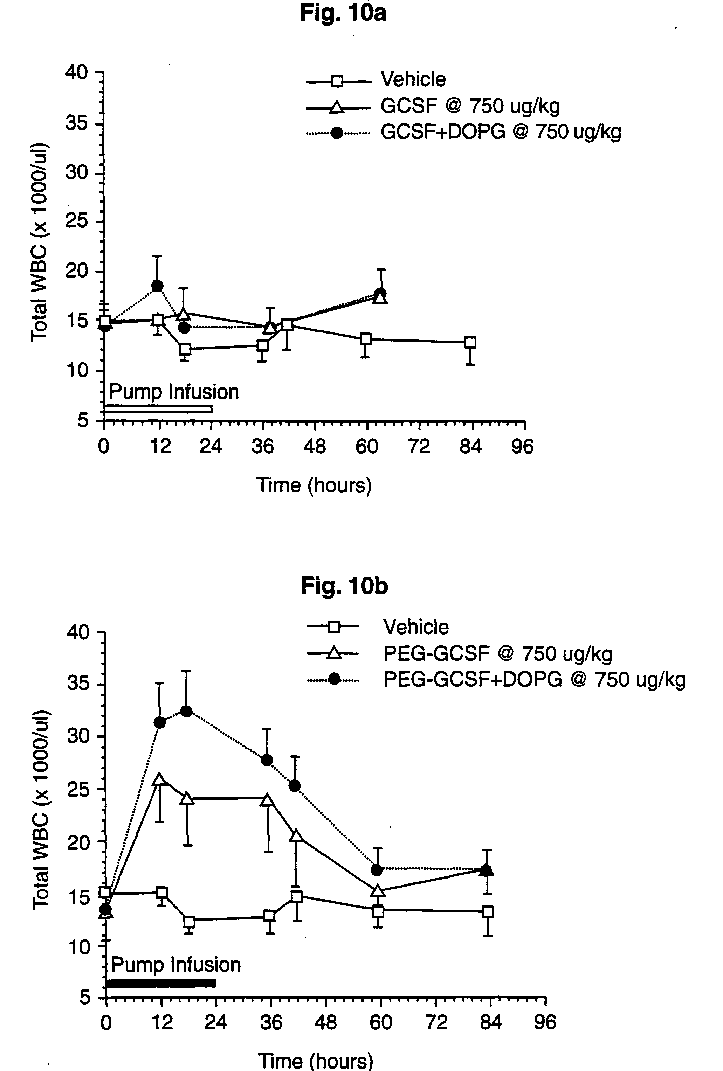

Methods. DOPG from Avanti Polar Lipids Inc., Alabaster Alabama, was dissolved in anhydrous chloroform to a final concentration of 100 mg/ml. 100 µmol of the lipid (797 µl) were dried under vacuum and then 1 ml of milli Q water was added to make a 100 mM solution of the lipid. This solution was sonicated for 5 minutes in a sonicating water bath (Model G 112SP1T from Laboratories Supply Inc., Hicksville, NY) or until the lipid solution was clear. 9 µmol of the DOPG solution (90 µl) were added to 90 nmol of rhG-CSF or PEG-G-CSF, prepared as described above, in 1 mM HCl. The solution was vortexed and brought to a final volume of 2 ml with 1 mM HCl, prior to loading into the Alzet osmotic pumps and implantation into the animals as previously described. Dosages are shown on FIGURE 10.

Results. The results are illustrated in FIGURES 10, showing white blood cell count effect, and 11, showing serum levels. For total white blood cell count, the use of PEG-G-CSF elicited a higher response even as compared to non-pegylated G-CSF + DOPG (comparing FIGURE 10(a) and FIGURE 10(b)). A comparison of PEG-G-CSF without DOPG, and PEG-G-CSF + DOPG, FIGURE 10(b) illustrates that DOPG enhances the biological effect, in terms of increased total white blood cell count, of PEG-G-CSF delivered to the gut. The PEG-G-CSF + DOPG increase was nearly two fold greater than for PEG-G-CSF alone.

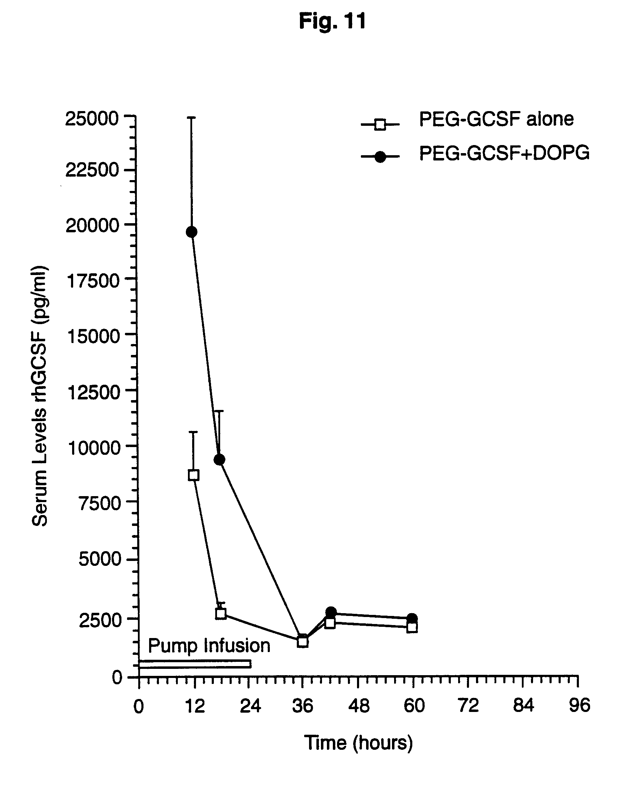

These results are confirmed by the serum levels of the protein, as shown in FIGURE 11. As illustrated, enteral infusion of PEG-G-CSF + DOPG results in at least a two fold increase in the serum levels of protein over PEG-G-CSF alone. The pharmacokinetics of the derivatived protein are unchanged, however.

These results demonstrate that use of an anionic lipid such as DOPG in an oral formulation of PEG-G-CSF increases the therapeutic response elicited by the derivatized protein. The increased response appears to be a result of greater bioavailability of the PEG-G-CSF.

**Table 12**

| Effect of Pegylation on the Enteral Bioavailability of Cytokine. | | | | |
|---|---|---|---|---|
| Dose Site | Protein | Dose (µg/kg) | AUC | Bio-availability (%) |
| IV | G-CSF | 25 | 200,000 | 100 % |
| ID | G-CSF | 755 | 0 | 0 % |
| ID | PEG-G-CSF | 823 | 630,000 | 9.45 % |

The PEG-G-CSF used above was a population of molecules wherein a majority contained at least three polyethylene glycol molecules attached thereto (see infra). In this way, the level of derivitization was similar to the more highly derivatized PEG-IFN-Con₁ (F1). The results in Table 12 show that these two derivatized proteins have similar bioavailability from the enteral route when they are compared to the unmodified protein infused intravenously. Therefore, a preferable form of a pegylated cytokine for enteral and therefore oral delivery, is a highly pegylated derivative.

In general, for pegylated G-CSF a much greater enteral bioavailability is demonstrated as compared to the enterally infused non-pegylated cytokine. Although the precise reason is not thoroughly understood, the increase in bioavailability could be due to the protease resistance of the pegylated form, the longer circulation time of the derivatized protein allowing it to accumulate in the body, an effect on the permeation of the protein across the enteral barrier, or a combination of these factors.

## Claims

1. An oral pharmaceutical composition for delivery to the gut, in particular to the small intestine, comprising a population of polyethylene glycol modified G-CSF molecules the majority of said population carrying per G-CSF molecule predominantly three and four polyethylene glycol moieties having an average molecular weight of about 6000.

2. The pharmaceutical composition according to claim 1, wherein the polyethylene glycol molecules are attached to an amino group of G-CSF.

3. The pharmaceutical composition according to claim 1, wherein the polyethylene glycol molecules are attached to a carboxyl group of G-CSF.

4. The pharmaceutical composition according to claim 1, wherein the polyethylene glycol molecules are attached to a sulfhydryl group of G-CSF.

5. The pharmaceutical composition according to claim 2, wherein the polyethylene glycol molecules are attached to the N-terminus of G-CSF.

6. The pharmaceutical composition according to claim 2, wherein the polyethylene glycol molecules are attached to a lysine group of G-CSF.

7. The pharmaceutical composition according to claim 1, wherein the polyethylene glycol molecules are attached at residues found in the external loops connecting alpha helices of the G-CSF.

8. The pharmaceutical composition according to any one of claims 1 to 7, wherein the pegylated G-CSF is associated with an anionic lipid.

9. The pharmaceutical composition according to claim 8, wherein the anionic lipid is dioleoyl phosphatidylglycerol.

10. The pharmaceutical composition according to any one of claims 1 to 9, wherein the G-CSF is recombinant human G-CSF.

11. The pharmaceutical composition according to any one of claims 1 to 10 comprising a pharmaceutically acceptable enteric coating for gastric juice resistance impermeable to at least pH 5.0.

## Patentansprüche

1. Orale pharmazeutische Zusammensetzung für eine Verabreichung an den Darm, insbesondere an den Dünndarm, umfassend eine Population von Polyethylenglykol-modifizierten G-CSF-Molekülen, wobei die Mehrheit der Population pro G-CSF-Molekül überwiegend drei und vier Polyethylenglykolreste mit einem durchschnittlichen Molekulargewicht von etwa 6000 trägt.

2. Pharmazeutische Zusammensetzung gemäß Anspruch 1, wobei die Polyethylenglykol-Moleküle an eine Aminogruppe von G-CSF gebunden sind.

3. Pharmazeutische Zusammensetzung gemäß Anspruch 1, wobei die Polyethylenglykol-Moleküle an eine Carboxylgruppe von G-CSF gebunden sind.

4. Pharmazeutische Zusammensetzung gemäß Anspruch 1, wobei die Polyethylenglykol-Moleküle an eine Sulfhydrylgruppe von G-CSF gebunden sind.

5. Pharmazeutische Zusammensetzung gemäß Anspruch 2, wobei die Polyethylenglykol-Moleküle an den N-Terminus von G-CSF gebunden sind.

6. Pharmazeutische Zusammensetzung gemäß Anspruch 2, wobei die Polyethylenglykol-Moleküle an eine Lysingruppe von G-CSF gebunden sind.

7. Pharmazeutische Zusammensetzung gemäß Anspruch 1, wobei die Polyethylenglykol-Moleküle an Reste gebunden sind, die sich in den externen Schleifen befinden, die alpha-Helices des G-CSF verbinden.

8. Pharmazeutische Zusammensetzung gemäß einem der Ansprüche 1 bis 7, wobei das pegylierte G-CSF mit einem anionischen Lipid assoziiert ist.

9. Pharmazeutische Zusammensetzung gemäß Anspruch 8, wobei das anionische Lipid Dioleoylphosphatidylglycerin ist.

10. Pharmazeutische Zusammensetzung gemäß einem der Ansprüche 1 bis 9, wobei das G-CSF rekombinantes menschliches G-CSF ist.

11. Pharmazeutische Zusammensetzung gemäß einem der Ansprüche 1 bis 10, umfassend eine pharmazeutisch verträgliche enterische Beschichtung für eine Resistenz gegenüber Magensaft, die bis wenigstens pH 5,0 undurchlässig ist.

## Revendications

1. Composition pharmaceutique orale destinée à être libérée dans l'intestin, en particulier, dans l'intestin grêle, comprenant une population de molécules G-CSF modifiées par un polyéthylène glycol, la majorité de ladite population portant, par molécule G-CSF, de manière prédominante, trois et quatre fragments de polyéthylène glycol ayant une masse moléculaire moyenne d'environ 6000.

2. Composition pharmaceutique selon la revendication 1, dans laquelle les molécules de polyéthylène glycol sont liées à un groupe amino du G-CSF.

3. Composition pharmaceutique selon la revendication 1, dans laquelle les molécules de polyéthylène glycol sont liées à un groupe carboxyle du G-CSF.

4. Composition pharmaceutique selon la revendication 1, dans laquelle les molécules de polyéthylène glycol sont liées à un groupe sulfhydryle du G-CSF.

5. Composition pharmaceutique selon la revendication 2, dans laquelle les molécules de polyéthylène glycol sont liées à l'extrémité N-terminale du G-CSF.

6. Composition pharmaceutique selon la revendication 2, dans laquelle les molécules de polyéthylène glycol sont liées à un groupe lysine du G-CSF.

7. Composition pharmaceutique selon la revendication 1, dans laquelle les molécules de polyéthylène glycol sont liées à des résidus qui se trouvent dans les boucles externes reliant les hélices alpha du G-CSF.

8. Composition pharmaceutique selon l'une quelconque des revendications 1 à 7, dans laquelle le G-CSF modifié par un PEG est associé à un lipide anionique.

9. Composition pharmaceutique selon la revendication 8, dans laquelle le lipide anionique est le dioléoyl-phosphatidylglycérol.

10. Composition pharmaceutique selon l'une quelconque des revendications 1 à 9, dans laquelle le G-CSF est le G-CSF humain recombinant.

11. Composition pharmaceutique selon l'une quelconque des revendications 1 à 10 comprenant un enrobage gastrorésistant et entérosoluble pharmaceutiquement acceptable imperméable jusqu'à au moins pH 5,0.
